(19) Europäisches Patentamt
European Patent Office
Office européen des brevets

(11) Veröffentlichungsnummer : **0 399 547 B1**

(12) # EUROPÄISCHE PATENTSCHRIFT

(45) Veröffentlichungstag der Patentschrift :
**11.08.93 Patentblatt 93/32**

(51) Int. Cl.$^5$ : **A61K 31/445**

(21) Anmeldenummer : **90109955.6**

(22) Anmeldetag : **25.05.90**

(54) **Verwendung von 4-(Hydroxydi-phenylmethyl)-1-piperidyl-phenylalkan-Derivaten zur Herstellung eines Antihistaminikums.**

(30) Priorität : **26.05.89 DE 3917241**

(43) Veröffentlichungstag der Anmeldung :
**28.11.90 Patentblatt 90/48**

(45) Bekanntmachung des Hinweises auf die Patenterteilung :
**11.08.93 Patentblatt 93/32**

(84) Benannte Vertragsstaaten :
**BE CH DE ES FR GB IT LI LU NL**

(56) Entgegenhaltungen :
**DE-A- 2 506 770**
**US-A- 3 014 037**
**CHEM. PHARM. BULL., Band 35, Nr. 11, 1987, Seiten 4637-4641; H. OHTAKA et al.: "Benzylpiperazine derivatives. VII. Studies on the role of the nitrogen atom in the cerebral vasodilating activity of 1-benzyl-4-diphenylmethylpiperazine derivatives"**

(73) Patentinhaber : **SCHAPER & BRÜMMER GMBH & CO. KG**
**Bahnhofstrasse 45**
**W-3320 Salzgitter 61 (DE)**

(72) Erfinder : **Pein, Eckhardt, Dr. med.**
**Sohnreystrasse 16**
**W-3410 Northeim (DE)**
Erfinder : **Ritter, Helmut, Prof., Dr.**
**Rotdornweg 37**
**W-5600 Wuppertal 1 (DE)**
Erfinder : **Laven, Reinhard, Dipl.-Ing.**
**Lindenstrasse 10**
**W-3320 Salzgitter 61 (DE)**

(74) Vertreter : **Lins, Edgar, Dipl.-Phys. Dr.jur. et al**
**Patentanwälte Gramm + Lins**
**Theodor-Heuss-Strasse 1**
**W-3300 Braunschweig (DE)**

EP 0 399 547 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Gegenstand der Erfindung ist die Verwendung von 4-(Hydroxydiphenylmethyl)-1-piperidyl-phenylalkan-Derivaten zur Herstellung eines Antihistaminikums.

Es ist bekannt, daß 4-Diphenyl-methyl-piperidin-Derivate als Antihistaminika verwendbar sind. Eine entsprechende Wirkung ist für 1-Piperidinobutanole und 1-Piperidinopropanole in den Schriften DE 25 06 770 A1, DE 23 03 305 A1, DE 23 03 306 A1, DE 25 03 362 A1 und DE 30 07 498 A1 beschrieben.

Eingang in die Therapie als Antihistaminikum hat das (α-[4-1,1-dimethylethyl)phenyl]-4(Hydroxydiphenylmethyl)-1-piperidin-butanol), INN; Terfenadin, gefunden.

Auf der Basis des αα-Diphenyl-4-piperidyl-methanols sind zahlreiche Substanzen, auch als pharmazeutisch wirksam, beschrieben worden. In US 3,014,037 und US 3,068,237 sind diese Substanzen als muskulotrop wirkende Spasmolytika vom Papaverintyp sowie als antisekretorisch wirksame Substanzen beschrieben. Weiterhin wurde die Verlängerung der Babiturat-Schlafzeit bei Säugetieren mitgeteilt.

In Chem. Pharm. Bull. 35, 4637 - 4641 (1987) wurde offenbart, daß α,α-Diphenyl-4-piperidyl-methanol-Derivate und chemisch nahestehende Verbindungen eine cerebral gefäßerweiternde Wirkung entfalten, d. h. daß sich die muskulotrop spasmolytische Wirkung in diesem Gefäßgebiet nachweisen ließ.

Erfindungsgemäß sind 4-(hydroxydiphenylmethyl)-1-Piperidylphenyl-alkan-Derivate zur Herstellung eines Arzneimittels für eine therapeutische Anwendung als $H_1$-Antihistaminikum verwendbar, wenn in der nachstehenden allgemeinen Strukturformel

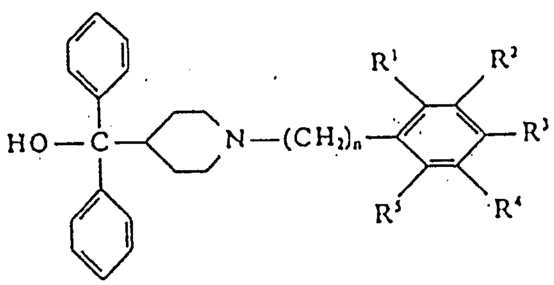

n 1 oder 2 ist und
$R^1$ - $R^5$ unabhängig voneinander für Wasserstoff und mindestens einer davon für linear oder verzweigte Alkylreste mit 1 - 4 C-Atomen, $C_1$ - $C_3$ Alkoxy- oder Hydroxyreste stehen.

Als Reste kommen insbesondere Methoxygruppen oder $C_1$ - $C_4$ Alkylgruppen in Frage, von denen der Phenylring vorzugsweise einen oder zwei aufweisen.

Der Nachweis der überraschenden und unerwarteten besonderen Wirkung der genannten 4-(Hydroxydiphenylmethyl)-1-piperidylphenylalkan-Derivate ergibt sich aus der pharmakologischen Wirkung der beschriebenen Verbindungen.

Beispielhaft für die Wirkungsweise der aufgezeigten Substanzgruppe ist die Wirkung des 4-(Hydroxydiphenylmethyl)-1-piperidyl -(3-methoxyphenyl)-methans.

Die Experimente wurden am isolierten Ileum sowie an der isolierten Trachea des Meerschweinchens in typischer Weise in vitro im geeigneten Organbad durchgeführt. Als Spasmodikum diente Histamin-Dihydrochlorid in einer Endkonzentration von 0,2 - 2 µg/ml, je nach Organpräparat. Die Aktivität der getesteten Substanzen wurde aus der reaktiven Spasmolyse, d. h. der relativen Abnahme der Kontraktionskraft, bezogen auf die Dosis Null, bestimmt. Die Konzentrationswirkungsbeziehung errechnete sich aus der nichtlinearen Regression zwischen Dosis und Wirkung aus mindestens 6 Einzelversuchen.

Aus genannten Untersuchungen ergab sich eine $ED_{50}$ am Ileum des Meerschweinchens von 62 ng/ml für Terfenadin sowie 29 ng/ml für die [4-(Hydroxydiphenylmethyl)-1-piperidyl]-(3-methoxyphenyl)methan-Verbindung. Am isolierten Trachealpräparat betrug die $ED_{50}$ für Terfenadin 4,1 µg/ml sowie 3,5, µg/ml für die [4-(Hydroxydiphenyl-methyl)-1-piperidyl]-(3-methoxyphenyl)-methan-Verbindung.

Insgesamt zeigten alle beschriebenen 4-(Hydroxydiphenylmethyl)-1-piperidyl-phenylalkan-Derivate eine vergleichbare Wirkungsstärke, wobei insgesamt die aufgezeigten Substanzen die Wirkung des Terfenadins aber übertrafen.

Erhebliche Wirkungsunterschiede zeigten sich unerwartet und überraschend bei der in vivo-Testung der beschriebenen 4-(Hydroxydiphenylmethyl)-1-piperidyl-phenylalkan-Derivate an der Maus nach oraler Applikation.

Die Experimente wurden an männlichen NMRI-Mäusen mit einem KGW von 25 - 30 g durchgeführt. Durch intradermale Injektion von 1µg Histamin wird die Kapillarpermeabilität artifiziell erhöht und dadurch die Freisetzung eines intravenös injizierten Farbstoffes ermöglicht. Als Maß für die Antihistamin-Aktivität kann die photometrisch bestimmte Farbstoffkonzentration eines definierten Hautareals bezeichnet werden.

In der Tabelle 1 ist die Beeinflussung der durch intradermale Injektion von 1 µg Histamin erzeugten Kapillarschädigung an der Rückenhaut der Maus dargestellt.

Dabei zeigte sich wiederum die überlegene Anthistaminwirkung der beanspruchten 4-(Hydroxydiphenylmethyl)-1-piperidyl-phenyl-alkan-Derivate im Vergleich zu dem Piperidinoalkohol-Derivat Terfenadin.

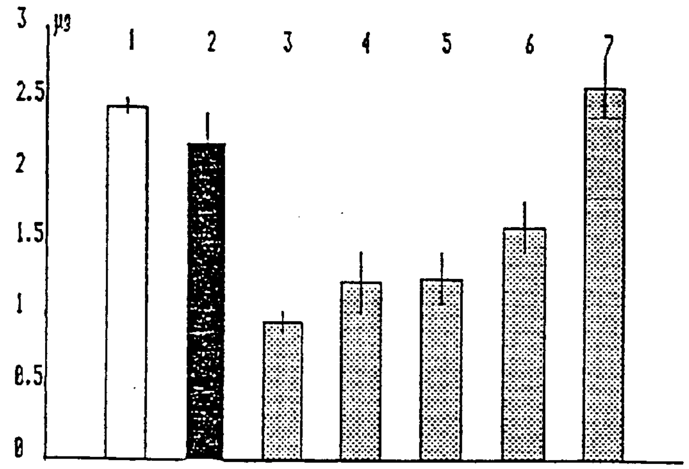


**Tabelle 1:** **Farbstoffgehalt der Hautareale an der Rückenhaut der Maus nach intradermaler Injektion von 1 µg Histamin nach Behandlung mit 1. physiologischer Kochsalzlösung, 2. Terfenadin 3 mg/kg, 3. 3 mg/kg 4-(Hydroxydiphenylmethyl)-1-piperidyl-(3-methylphenyl)-methan HCl 4. 3 mg/kg 4-(Hydroxydiphenylmethyl)-1-piperidyl-(4-methoxyphenyl)-ethan HCl 5. 3 mg/kg 4-(Hydroxydiphenylmethyl)-1-piperidyl-(3-methoxyphenyl)-ethan HCl 6. 3 mg/kg 4-(Hydroxydiphenylmethyl)-1-piperidyl-(4tert.-butyl-phenyl)-methan HCl. 7. 6 mg/kg 4-(Hydroxydiphenyl-methyl)-1-piperidyl-methan**

Bei der Abklärung des pharmakologischen Wirkprofils der α,α-Diphenylpiperidylphenylalkan-Derivate zeigte sich, daß die Antihistaminwirkung für die aufgezeigten Substanzen um den Faktor 2 - 3 stärker ausgeprägt war als die muskulotrop-spasmolytische Wirkung, d. h. daß die Antihistaminwirkung die dominierende pharmakodynamische Eigenschaft der beanspruchten α,α-Diphenylpiperidylphenylalkan-Derivate darstellt.

Die neu entdeckte und unerwartete Wirkungsqualität der aufgezeigten Substanzklasse bedeutet in der Therapie allergischer Erkrankungen gegenüber den Piperidinoalkoholderivaten vom Terfenadintyp, daß mit einer wesentlichen geringeren individuellen Dosis und deshalb auch mit geringeren unerwünschten Wirkungen bei der Therapie zu rechnen ist.

Die ausgeprägte Affinität der 4-(Hydroxydiphenylmethyl)-1-piperidylphenylalkan-Derivate zu den $H_1$-Histamin-Rezeptoren der Haut läßt sich auch eine therapeutische Wirksamkeit noch in den Konzentrationen erwarten, wo entsprechende Piperidinoalkoholderivate keine ausreichende Wirkung entfalten.

Die aufgezeigten 4-(Hydroxydiphenylmethyl)-1-piperidyl-phenylalkan-Derivate können in reiner Form oder in üblichen galenischen Formulierungen sowohl oral, parenteral als auch rektal appliziert sowie in Aerosol-

oder Pulverform verabreicht werden.

Der Anwendungsbereich der beschriebenen 4-(Hydroxydiphenylmethyl)-1-piperidyl-phenylalkan-Derivate als Antihistaminika erstreckt sich sowohl auf den human- als auch den gesamten veterinärmedizinischen Bereich.

Auffallend war, daß die in vivo-Wirksamkeit mit zunehmender Zahl der C-Atome für n deutlich abnahm und bei n = 3 sich dem schwächeren Wirkungsniveau des Terfenadins näherte.

Bei der Untersuchung zur Wirkungsdauer der 4-(Hydroxydiphenylmethyl)-1-piperidyl-phenylalkan-Derivate zeigte sich zusätzlich und unerwartet, daß die erfindungsgemäßen Substanzen nach einmaliger oraler Gabe von 6 mg/kg KGW bis zu über 6 Stunden eine deutliche Hemmung der Histamin-induzierten Hautquaddel an der Maus besaßen. Bei der Vergleichssubstanz Terfenadin war dagegen nach 4 Stunden keine sichere Antihistaminwirkung nach oraler Applikation in vergleichbarer Dosis mehr objektivierbar.

Die experimentellen Ergebnisse belegen, daß die beschriebenen 4-(Hydroxydiphenylmethyl)-1-piperidylphenylalkan-Derivate effektive Antihistaminika, Antiallergika und Bronchodilatatoren darstellen, die eine wirksame Behandlung entsprechender Krankheitszustände erwarten lassen.

Die Erfindung umfaßt pharmazeutisch zulässige Salze mit genannten 4-(Hydroxydiphenylmethyl)-1-piperidyl-phenylalkan-Derivaten. Pharmazeutisch zulässige Säureadditionssalze sind solche mit geeigneten organischen und anorganischen Säuren wie Salzsäure, Schwefelsäure, Phosphorsäure oder Carbonsäuren und Dicarbonsäuren. Zu den geeigneten organischen Säuren gehören Essigsäure, Fettsäuren, wie Stearinsäure, Laurinsäure, Ölsäure oder Palmitinsäure, Glycolsäure, Milchsäure, Brenztraubensäure, Malonsäure, Bernsteinsäure, Fumarsäure, Äpfelsäure, Weinsäure, Zitronensäure, Cyclamensäure, Ascorbinsäure, Benzoesäure, 4-Hydroxybenzoesäure, Zimtsäure, Salicylsäure, Mandelsäure sowie die geeigneten Sulfonsäuren wie Methansulfonsäure, Äthansulfonsäure und ß-Hydroxyethansulfonsäure.

Die Salze werden auf konventionelle Weise gebildet.

Beispiele für erfindungsgemäße Verbindungen sind:

1. [4-(Hydroxydiphenylmethyl)-1-piperidyl]-(3-methoxyphenyl)-methan.
2. [4-(Hydroxydiphenylmethyl)-1-piperidyl]-(3-methoxyphenyl)-ethan.
3. 1-[4-(Hydroxydiphenylmethyl)-1-piperidyl)]-2-(3-methoxyphenyl-ethan.
4. 4-(Hydroxydiphenylmethyl)-1-piperidyl-(4-tert. butylphenyl)-methan.

Die erfindungsgemäßen Derivate des 4-(Hydroxydiphenylmethyl)-1-piperidyl-phenylalkans können oral, parenteral, z. B. intravenös, subcutan, intramusculär, intranasal oder rektal appliziert sowie in Nebel- oder Pulverform verabreicht werden.

Die Dosen der applizierten Substanz hängen von der Art der Anwendung und der individuellen Empfindlichkeit des zu Therapierenden ab. Der Dosisbereich erstreckt sich von 0,1 - 10 mg/kg KGW/Tag zur Erzielung der erwünschten Wirkung.

Die erfindungsgemäßen Substanzen können auf unterschiedliche Weise hergestellt werden, wie anhand der nachstehenden Synthesebeispiele für vier beispielhafte Produkte gezeigt wird:

1. Synthese von 1-[4-(Hydroxydiphenylmethyl)-1-piperidyl]-2-(4-methoxyphenyl)-ethan

In einem 250 ml Rundkolben wird eine Mischung aus 4 g α,α-Diphenyl-4-piperidinomethanol, 0,94 g pulverisiertes Kaliumhydroxid, 200 mg KJ in 50 ml Toluol dispergiert. Nach Zugabe von 2,3 ml 4-Methoxyphenylethylchlorid wird 3 Tage unter Rückfluß erhitzt.

Nach Abkühlen auf Raumtemperatur wird von der Trübung abfiltriert, das Lösungsmittel abdestilliert, der Rückstand in 50 ml Chloroform gelöst und mit Wasser mehrfach ausgeschüttelt. Die organische Phase wird anschließend mit $MgSO_4$ getrocknet, das Lösungsmittel abdestilliert und der Rückstand mit Ether überschichtet, wobei ein weißes kristallines Produkt resultiert.

Zur weiteren Reinigung wird das Rohprodukt in Methylenchlorid/Essigsäureethylester (1:1) gelöst und mittels einer mit Kieselgel gefüllten Säule (4 cm Laufhöhe) von geringen Verunreinigungen befreit.

Nach Abdestillieren des Lösungsmittels resultiert ein farbloses, kristallines Produkt. Schmelzpunkt 120° C Charakteristische IR-Banden: 1608, 1505, 1440, 1240, 705 $cm^{-1}$

2. 4-(Hydroxydiphenylmethyl)-1-piperidyl)-(3-methoxyphenyl)-methan-hydrochlorid

Ein Gemisch aus 8,0 g (30 mmol) α,α-Diphenyl-4-piperidinomethanol 2,35 g (15 mmol) 3-Methoxybenzylchlorid, 0,5 g Triethylamin und 30 ml THF wird 1 Stunde unter Rückfluß gerührt, wobei ein farbloser Niederschlag ausfällt. Nach Abkühlen auf 20° C wird der Niederschlag abfiltriert, das Filtrat eingeengt. Zur Aufarbeitung wird der Rückstand in 5 ml Methylenchlorid aufgenommen und solange mit etherischer HCL-Lösung versetzt, bis kein Niederschlag mehr auftritt. Das Gemisch wird mit 30 ml Methylenchlorid verdünnt,

der Niederschlag filtriert und das Filtrat eingeengt. Man erhält nach dem Trocknen des öligen Rückstandes bei 40° C im Vakuum 6,5 g Endprodukt.
Zersetzungspunkt ca. 80° C
Charakteristische IR-Banden: 3360, 1590, 1480, 1438, 1256, 690 $cm^{-1}$

3. Synthese von 1-[4-(Hydroxydiphenylmethyl)-1-piperidyl)]-2-3-methoxyphenyl)-ethan

Zu einem Gemisch aus 5,78 g (21,7 mmol) $\alpha,\alpha$-Diphenyl-4-piperidinomethanol, 2,2 g Thriethylamin in 20 ml absolutem THF werden unter Rühren bei 0° C unter Stickstoff 2,0 g (10,8 mmol) 3-Methoxyessigsäurechlorid zugetropft. Nach vierstündigem Rühren bei Raumtemperatur wird das Gemisch in 20 ml Chloroform aufgenommen, der Niederschlag filtriert und das Filtrat bis zur Trockne eingedampft. Der Rückstand wird in möglichst wenig Methylenchlorid/Essigester (1 : 1,Vol.) gelöst und über eine mit Kieselgel gefüllte Säule (Höhe 7 cm, Durchmesser 3 cm) mit Essigester als Laufmittel gereinigt. Nach Einengen und Trocknen erhält man 2,5 g Produkt, welches im IR-Spektrum eine charakteristische Amidbande bei 1630 $cm^{-1}$ ergibt.

In 20 ml THF, welches über LiA1H, getrocknet wurde, werden 0,25 g LiA1H und anschließend 2,5 g des Produktes aus der ersten Stufe zugegeben. Es wird 2 Stunden bei Raumtemperatur gerührt, wobei im Abstand von 30 Minuten jeweils ca. 100 mg LiA1H, nachdosiert werden. Anschließend wird noch eine weitere Stunde unter Rückfluß erwärmt. Nach dem Abkühlen wird das LiA1H vorsichtig mit wenig Wasser zersetzt und dem Gemisch solange $Al_2O_3$ zugegeben, bis eine klare überstehende Lösung resultiert. Nach Filtration und Abdestillieren des Lösungsmittels wird der ölige Rückstand solange mit etherischer HCl-Lösung versetzt, bis kein Niederschlag mehr resultiert. Nach Zugabe von 50 ml Ether wird das Endprodukt abfiltriert und getrocknet. Die Ausbeute beträgt 1,7 g eines farblosen Produktes. Fp.: > 80° C (Zersetzung), Charakteristische IR-Signale: 3370 (OH), 1595 (Aromat), 1255, 1150, 745 und 695 $cm^{-1}$.

4. Synthese von 4-(Hydroxydiphenylmethyl)-1-piperidyl-(4-tert. butylphenyl)-methan

2,4 ml 4-tert.-Butylbenzylbromid, 7 g $\alpha,\alpha$-Diphenyl-4-piperidinomethanol und 1,9 ml Triethylamin werden in 90 ml absolutem THF gelöst und 1 Std. unter Rückfluß erhitzt. Danach wird filtriert und das Filtrat bis zur öligen Konsistenz eingeengt. Anschließend wird erneut in 50 ml Ether aufgenommen, mit der gleichen Menge Petrolether versetzt und nach Abkühlen filtriert. Nach Einengen wird das Öl mit wenig Ether gemischt und die Lösung mit etherischer HCl-Lösung zur Bildung des Hydrochlorids versetzt. Ausbeute: 3,5 g Schmp.: 110° C (Zersetzung). Charakteristische IR-Daten: 3300 (OH), 2960 (CH), 1615/1600 (Aromaten), 1450, 750 und 710 $cm^{-1}$ (KBr)

## Patentansprüche

1. Verwendung von 4-(Hydroxydiphenylmethyl)-1-piperidylphenylalkan-Derivaten der Formel

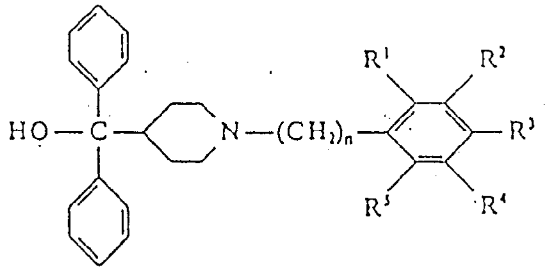

worin
n eine ganze Zahl zwischen 1 und 2,
$R_1$ bis $R_5$ unabhängig voneinander für Wasserstoff und mindestens einer davon für lineare oder verzweigte Alkylreste mit 1 - 4 C-Atomen, $C_1$ - $C_3$ Alkoxy- oder Hydroxy-Reste stehen, zur Herstellung eines Arzneimittels für eine therapeutische Anwendung als $H_1$-Antihistaminikum.

2. Verwendung von Derivaten nach Anspruch 1, bei denen einer oder zwei der Reste $R_1$ - $R_5$ Methoxygruppen darstellen.

3. Verwendung von Derivaten nach Anspruch 1, bei denen einer oder zwei der Reste $R_1$ - $R_5$ für $C_1$ - $C_4$ Alkylgruppen stehen.

4. Verwendung eines pharmazeutisch verträglichen Salzes eines Derivats nach einem der Ansprüche 1 bis 3.

## Claims

1. Use of 4-(hydroxydiphenylmethyl)-1-piperidyl-phenylalkane derivatives of the formula

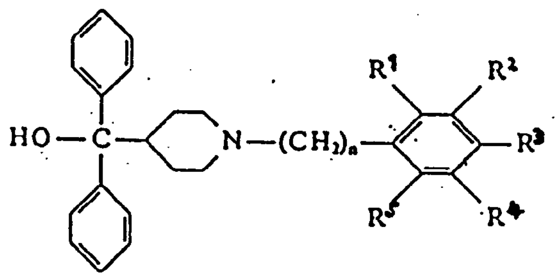

where
n stands for a whole number between 1 and 2,
$R_1$ to $R_5$ are mutually independent and stand for hydrogen and at least one of them stands for linear or branched alkyl radicals with 1 to 4 C atoms, $C_1$-$C_3$ alkoxy radicals or hydroxyl radicals, for preparing a medicament for therapeutic use as an $H_1$ antihistamine.

2. Use of derivatives according to Claim 1, in which one or two of the radicals $R_1$ to $R_5$ are methoxy groups.

3. Use of derivatives according to Claim 1, in which one or two of the radicals $R_1$ to $R_5$ stand for $C_1$-$C_4$-alkyl groups.

4. Use of a pharmaceutically acceptable salt of a derivative according to one of Claims 1 to 3.

## Revendications

1. Utilisation de dérivés de 4-(hydroxydiphénylméthyl)-1-pipéridylphénylalcane de formule :

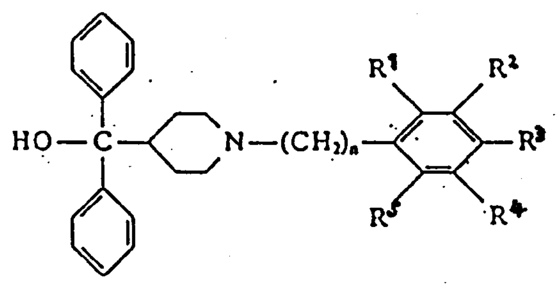

dans laquelle
n représente un nombre entier parmi 1 et 2,
$R^1$ - $R^5$ représentent indépendamment l'un de l'autre un hydrogène et au moins un d'entre eux représente des radicaux alcoyle en $C_1$ à $C_4$linéaires ou ramifiés, des radicaux alcoxy en $C_1$ à $C_3$ ou hydroxy, pour la préparation d'un médicament aux fins d'application thérapeutique comme antihistaminique $H_1$ .

2. Utilisation de dérivés selon la revendication 1, dans lesquels un ou deux des radicaux $R^1$ - $R^5$ représente(nt) un(des) groupe(s) méthoxy.

3. Utilisation de dérivés selon la revendication 1, dans lesquels un ou deux des radicaux $R^1$ - $R^5$ représente(nt) un(des) groupe(s) alcoyle en $C_1$ à $C_4$.

4. Utilisation d'un sel pharmaceutiquement acceptable d'un dérivé selon l'une des revendications 1 à 3.